# EUROPEAN PATENT SPECIFICATION

(11) **EP 2 536 829 B1**
(45) Date of publication and mention of the grant of the patent: **06.04.2016**
(21) Application number: 11703221.9
(22) Date of filing: 14.02.2011
(51) Int. Cl.: C12N 15/86, C12N 7/00, C12M 3/06

(54) **Method for the production of Ad26 adenoviral vectors**
Verfahren zur Herstellung von adenoviralen Ad26-Vektoren
Procédé de production de vecteurs adénoviraux Ad26

(30) Priority: 15.02.2010 US 304553 P; 15.02.2010 EP 10153581
(43) Date of publication of application: 26.12.2012
(73) Proprietor: Crucell Holland B.V., 2333 CN Leiden (NL)
(72) Inventor: LUITJENS, Alfred, NL-2333 CN Leiden (NL); VAN HERK, Herman, NL-2333 CN Leiden (NL)
(74) Representative: Beslier, Victor
(86) International application number: PCT/EP2011/052109
(87) International publication number: WO 2011/098592

(56) References cited:
- WO-A1-00/70071
- WO-A1-2004/055187
- WO-A1-2010/060719
- WO-A2-2007/104792
- YUK INN H Y ET AL: "Perfusion cultures of human tumor cells: A scalable production platform for oncolytic adenoviral vectors", 20 June 2004 (2004-06-20), BIOTECHNOLOGY AND BIOENGINEERING, WILEY & SONS, HOBOKEN, NJ, US LNKD- DOI:10.1002/BIT.20158, PAGE(S) 637 - 642, XP002496481, ISSN: 0006-3592 the whole document
- SUBRAMANIAN S ET AL: "Pilot-scale adenovirus seed production through concurrent virus release and concentration by hollow fiber filtration", BIOTECHNOLOGY PROGRESS, AMERICAN INSTITUTE OF CHEMICAL ENGINEERS, US LNKD- DOI:10.1021/BP049561A, vol. 21, no. 3, 1 May 2005 (2005-05-01), pages 851-859, XP002516027, ISSN: 8756-7938 [retrieved on 2005-03-30]
- XIE LIANGZHI ET AL: "Large-scale propagation of a replication-defective adenovirus vector in stirred-tank bioreactor PER.C6TM cell culture under sparging conditions", 5 July 2003 (2003-07-05), BIOTECHNOLOGY AND BIOENGINEERING, WILEY & SONS, HOBOKEN, NJ, US LNKD- DOI:10.1002/BIT.10644, PAGE(S) 45 - 52, XP002296365, ISSN: 0006-3592 the whole document
- KAMEN A ET AL: "Development and optimization of an adenovirus production proces", 1 January 2004 (2004-01-01), HUMAN GENE THERAPY, MARY ANN LIEBERT, NEW YORK ,NY, US, PAGE(S) S184 - S192, XP002486824, ISSN: 1043-0342 the whole document
- ABBINK PETER ET AL: "Comparative seroprevalence and immunogenicity of six rare serotype recombinant adenovirus vaccine vectors from subgroups B and D", 1 May 2007 (2007-05-01), JOURNAL OF VIROLOGY, THE AMERICAN SOCIETY FOR MICROBIOLOGY, US, PAGE(S) 4654 - 4663, XP002451979, ISSN: 0022-538X the whole document
- YALLOP C ET AL: "Per.C6 cells for the manufacture of biopharmaceutical proteins", MODERN BIOPHARMACEUTICALS : DESIGN, DEVELOPMENT AND OPTIMIZATION, WILEY-VCH, WEINHEIM, vol. 3, 1 January 2005 (2005-01-01), pages 779-807, XP008102484, ISBN: 978-3-527-31184-2
- ALTARAS N E ET AL: "Production and Formulation of Adenovirus Vectors", 1 November 2005 (2005-11-01), ADVANCES IN BIOCHEMICAL ENGINEERING, BIOTECHNOLOGY, SPRINGER, BERLIN, DE LNKD- DOI:10.1007/10_008, PAGE(S) 193 - 260, XP009105897, ISSN: 0724-6145 the whole document
- HENRY OLIVIER ET AL: "Insights into adenoviral vector production kinetics in acoustic filter-based perfusion cultures", 30 June 2004 (2004-06-30), BIOTECHNOLOGY AND BIOENGINEERING, WILEY & SONS, HOBOKEN, NJ, US LNKD- DOI:10.1002/BIT.20074, PAGE(S) 765 - 774, XP008102488, ISSN: 0006-3592 [retrieved on 2004-05-10] the whole document

## Description

The invention relates to the field of cell culture and adenovirus production. More particularly, it concerns improved methods for the culturing of mammalian cells, infection of those cells with adenovirus and the production of adenovirus particles therefrom.

### Background of the invention

Recent developments in the field of DNA vaccination using recombinant viral vectors have created the need for large scale manufacturing of clinical grade material. Processes are needed to be able to support the less and least developed world with sufficient amounts of recombinant adeno-based vaccines to fight e.g. the Tuberculosis and Malaria problem in the world. An evaluation of the birth cohort shows that more than 150.000.000 births are expected for the less and least developed world in 2010-2015. Based on this birth cohort the projected annual demand for a vaccine could reach approximately 1.5 x 10¹⁹ virus particles (VP) on a yearly basis (http://esa.un.org/unpp/index.asp?panel=2).

Several processes for production of adenoviruses have been described: These processes use adherent cell cultures in roller bottles, cell factories (Nunclon from Nunc or CellStack from Corning), or Cell Cubes (Corning). Production processes on adherent cell cultures cannot fulfill the worldwide demand for adeno-based vaccines. Therefore the cells used in the adherent process are adapted to suspension cultures (e.g. HEK293 and PER.C6^{®} cell lines). With the use of suspension cultures it is possible to scale-up production processes to large-scale bioreactors. Suspension cell cultures for adenovirus production are routinely achieved between 3 to 20L scale and successful scale-up has been reported up to 100L (Kamen et al., 2004), and 250L (Xie et al., 2003). Experiments are reported in which scaling up to 10.000L is anticipated (Xie et al., 2003).

However, a major disadvantage of scaling up to 10.000L is the high capital investment (CAPEX), which is needed to design and build a 10.000L bioreactor facility. Furthermore, the CAPEX commitment of building a 10.000L facility, under BSL 2 conditions, must be realized before even knowing if the product will be successful (Phase IV and beyond). The total investment cost for a 10.000L bioreactor plant is reported between €225.000.000 and €320.000.000 (Estape et al., 2006). Therefore, preparation at lower scale, e.g. in 1000L or smaller bioreactors, would be desirable.

With the use of currently existing processes, more than 150 batches at 1000L scale a year must be produced in order to reach the target of 1.5xl0¹⁹VP/year. Therefore, a need exists to improve systems for adenovirus production, to improve yields of adenovirus particles in order to fulfil the world-wide demand of adenovirus vaccines, preferably at non-prohibitive costs.

One of the issues encountered in adenovirus production optimization is the so-called "cell density effect". In batch-mode operation, several references suggest the existence of an optimal cell density at infection for adenovirus production. The optimum lies between 0.5 - 1x10⁶ cells/mL (Maranga et al., 2005; Kamen et al., 2004). It was shown for adenovirus (Ad5) production in a batch stirred tank bioreactor that the virus productivity per cell remains constant up to around 0.9x10⁶ cells/mL, but drops abruptly at around 1x10⁶ cells/mL (Altaras et al., 2005). Beyond 2x10⁶ cells/mL, no infectious particles were detectable. The breakpoint related to specific production drop with cell densities at infection is medium dependent. No available commercial medium to date has shown potential to support high yields of virus particles, while maintaining the specific production optimal at cell densities beyond 1x10⁶ cells/mL (Kamen et al., 2004). The reasons for this drop is not known yet but might be due to limited nutrient availability for virus production, or due to high metabolites concentrations that are inhibitory for virus production.

Fed-Batch operations, like addition of glucose, glutamine and amino acids allowed infections at cell densities up to 2x10⁶ cells/mL. However, the productivities attained at high cell densities were lower than those obtained with infection at cell densities of 1x10⁶ cells/mL (Kamen et al., 2004).

In perfusion processes the cells are retained in the bioreactor by hollow fibers, spin filters or acoustic separators while culture medium is perfused through the bioreactor. In these processes cell densities of >100x10⁶ cells/mL can sometimes be reached (e.g., Yallop et al., 2005).

Infected perfusion cells showed premature cell loss during perfusion with a hollow fiber system. This might be related to their higher shear sensitivity due to the viral infection (Cortin et al., 2004). The hydro-dynamical stresses induced in the tubing, the hollow fibers, or the peristaltic pump on more fragile, infected cells was most likely the cause for this phenomenon. Since infected cells are more fragile, particularly the acoustic separator (Henry et al., 2004) has been suggested to be desirable if the perfusion is to be maintained throughout the infection phase. However, infections performed in perfusion mode could only be maintained for cell densities up to 3x10⁶ cells/mL with a perfusion rate of 2 vol/day. Infection at a cell density of 6x10⁶ cells/mL led to a fivefold reduction in specific productivity (Henry et al., 2004).

Despite the reported cell density effect by others, one report (Yuk et al, 2004) described successful perfusion cultures of human tumor cells as a production platform for oncolytic adenoviral vectors. That report described a high-cell-density perfusion process using alternating tangential flow (ATF) technology. At an average viable cell density at infection of 9 x 10⁶ HeLaS3 cells/mL, an average viral titer of about 4 x 10¹¹ VP/mL was observed. The tumor cells used in that report are not preferred as production cells, since use of tumor cells may pose safety risks when the produced adenovirus particles are to be administered to humans. The recombinant adenovirus in that report was based on Ad5. Such adenoviruses have limited possibilities for use as vaccines since a majority of the human population contain pre-existing neutralizing antibodies against Ad5, and recombinant adenoviruses from other serotypes are therefore more suitable for use as vaccines (see e.g. WO 00/70071). Recombinant adenoviruses especially advantageous for use as vaccines include Ad26 (WO 00/70071).

WO2007/104792 discloses the production of rAd26 recombinant vector, similar to the production of the other adenoviral vectors derived from rAd5, rAd11, rAd35, rAd50, rAd48 and rAd49, wherein in PER.C6 producer cells are infected by rAd26 at a cell density between 10 x 10⁶ viable cells/ml and 16 x 10⁶ viable cells/ml when cultured in suspension with a perfusion system.

Limited information, if any, is available for the large scale production of recombinant adenoviruses from other serotypes than Ad5, in particular for the advantageous serotype 26. Some differences between Ad35 and Ad5 production at large scale have been described previously in e.g. PCT/EP2009/064265. The somewhat different physical properties of recombinant adenoviruses of different serotypes may give rise to differences in production processes. Such potential differences may especially be important at industrial scale, where even seemingly small differences at small scale may have large economic consequences on the scale envisaged for production of the annual world-wide demand. For instance, it was shown by the applicant that the reported cell density effect for Ad5 was different for Ad35 (PCT/EP2009/064265). Thus, rAd35 propagates differently in producer cells than rAd5 during large scale production processes. Apparently, the propagation of adenoviruses from different serotypes is very unpredictable.

In order to fulfil the world-wide demand of recombinant adenovirus serotype 26 (rAd26) vaccines, a need exists to improve systems for rAd26 production. The present invention provides improved methods for industrial production of rAd26.

### Summary of the invention

We have found herein that yet another serotype, i.e. Ad26 behaves differently than other serotypes Ad5 and Ad35. Indeed, Ad26 tends to show a slight cell density effect, yet not as accentuated as the density effect seen for Ad5. In addition, cells that are infected with Ad26 tend to grow further after infection, while cells infected with Ad35 show a decreased growth post infection.

These results again suggest that processes for specific adenovirus serotypes may have to be fine-tuned for each serotype, in order to obtain optimal results. The present invention provides an optimized system for production of rAd26 in terms of yield, quality of the rAd26 obtained, and ease of handling of the harvest for down stream processing.

The invention provides a method for producing recombinant adenovirus serotype 26 (rAd26), the method comprising: a) culturing PER.C6 cells in suspension with a perfusion system; b) infecting said cells at a density of between about 10x10⁶ viable cells/mL and 16x10⁶ viable cells/mL with rAd26; c) further culturing the infected cells with a perfusion system to propagate said rAd26; and d) harvesting said rAd26.

In certain embodiments said cells in step b) are infected with rAd26 at a density of between about 10x10⁶ and 14x10⁶ viable cells/mL.

In certain preferred embodiments, said perfusion system in step c) is an alternating tangential flow (ATF) perfusion system. In other preferred embodiments, said perfusion system in step a) is an alternating tangential flow (ATF) perfusion system. In a preferred aspect, said perfusion system in both steps a) and c) is an alternating tangential flow (ATF) perfusion system.

In certain embodiments, the method of the invention further comprises: e) purifying the rAd26. In further embodiments, the method further comprises: f) preparing a pharmaceutical composition containing the purified rAd26.

In certain embodiments, said recombinant adenovirus lacks at least a portion of the E1 region, and comprises heterologous nucleic acid.

In certain embodiments, the step a) is performed in a first bioreactor, and steps b) and c) are performed in a second bioreactor.

In preferred embodiments, the physical particle to infectious particle (VP/IU) ratio of the produced rAd26 is less than 30:1, preferably less than 20:1.

It is also an aspect of the invention to provide a method for producing at least 1x10¹² rAd26 virus particles (VP)/mL, the method comprising: a) culturing PER.C6 cells in suspension with a perfusion system; b) infecting said cells at a density of between about 10x10⁶ viable cells/mL and 16x10⁶ viable cells/mL with rAd26; c) further culturing the infected cells with a perfusion system to propagate said rAd26, whereby the concentration of rAd26 virus particles reaches at least 1x10¹² VP/mL; and d) harvesting said rAd26.

The invention also provides use of a bioreactor in a method according to the invention, wherein the bioreactor has a working volume of between 2L and 1000L, comprising: culture medium, PER.C6 cells, and at least 1x10¹² rAd26 virus particles (VP)/mL. In a preferred aspet, the bioreactor has a working volume of between 50L and 500L. In preferred embodiments, the bioreactor is connected to an ATF perfusion system. In preferred embodiments the rAd26 virus particle have a VP/IU ratio of less than 30:1, preferably less than 20:1.

### Brief description of the Figures

FIG. 1. Infection at high cell density in shakers with rAd5.
FIG. 2. Infection at high cell density in shakers and 2L bioreactor with rAd35.TB-S.
FIG. 3. Infection at high cell density in shakers with rAd26.
FIG. 4. Cell growth post infection with rAd26.
FIG. 5. Cell growth post infection with rAd35.

### Detailed description of the invention

The present invention describes a new process for the production of large quantities of recombinant adenovirus rAd26. This optimized process relies on the ability to infect cultures at high cell density with preservation of a high virus productivity per cell. Herewith, it offers a method to obtain a harvested virus solution with high virus concentration in a single bioreactor. Typical yields of current processes for rAd26 are about 2-3x10¹¹ VP/mL. Indeed, it is believed that very large quantities of rAd26 particles can be produced using the processes of the present invention, for instance quantities of at least about 5x10¹¹ VP/mL, preferably at least about 6, 7, 8, or 9x10¹¹ VP/mL. Preferably at least 1x10¹² VP/mL of rAd26 are produced, more preferably at least 1.5x10¹² VP/mL, still more preferably at least 2x10¹² VP/mL, e.g. between about 1x10¹² and 5x10¹² VP/mL. Typically, the process will not yield more than about 1x10¹³ VP/mL of rAd26. The yields that can be obtained with the processes according to the present invention, are likely sufficient to prepare the desired amount of certain rAd26 based vaccines in the world, without requiring bioreactor facilities with working volumes larger than 1000L.

The invention provides a method for producing recombinant adenovirus serotype 26 (rAd26), the method comprising: a) culturing PER.C6 cells in suspension with a perfusion system; b) infecting said cells at a density of at least 10x10⁶ viable cells/mL with rAd26; c) further culturing the infected cells with a perfusion system to propagate said rAd26; and d) harvesting said rAd26.

In certain embodiments, said cells in step b) are infected with rAd26 at a density of between about 10x10⁶ and 50x10⁶ viable cells/mL. In further embodiments, said cells in step b) are infected with rAd26 at a density of between about 10x10⁶ and 20x10⁶ viable cells/mL. In yet further advantageous embodiments, said cells in step b) are infected with rAd26 at a density of between about 10x10⁶ and 16x10⁶ viable cells/mL, for instance at about 10, 11, 12, 13, 14 or 15x10⁶ viable cells/mL.

### Producer Cells and Recombinant Adenovirus

A producer cell (sometimes also referred to in the art and herein as 'packaging cell' or 'complementing cell' or 'host cell') according to the present invention is a PER.C6 cell wherein a desired adenovirus can be propagated. For example, the propagation of recombinant adenovirus vectors is done in producer cells that complement deficiencies in the adenovirus. Such producer cells preferably have in their genome at least an adenovirus E1 sequence, and thereby are capable of complementing recombinant adenoviruses with a deletion in the E1 region. Further the adenovirus may have a deletion in the E3 region, which is dispensable from the Ad genome, and hence such a deletion does not have to be complemented. E1-complementing PER.C6 cells can be used, which are human retina cells immortalized by E1 (see US patent 5,994,128). PER.C6 cells (as deposited on 29 February 1996 under number ECACC deposit 96022940 at the European Collection of Cell Cultures, CAMR, Salisbury, Wiltshire, UK; see US patent 5,994,128), or cells derived therefrom are used as producer cells.

It is shown herein that recombinant adenovirus serotype 35 (rAd35) has hitherto unknown, advantageous, properties compared to rAd5 in processes applying high cell density infection. We now also know that yet another serotype (rAd26) again behaves differently in similar processes than the two previously mentioned serotypes, suggesting that optimal conditions for large scale production of recombinant adenovirus may have to be established for different serotypes. The adenovirus of the present invention is rAd26.

Preferably, the adenoviral vector is deficient in at least one essential gene function of the E1 region, e.g., the E1a region and/or the E1b region, of the adenoviral genome that is required for viral replication. In certain embodiments, the vector is deficient in at least one essential gene function of the E1 region and at least part of the nonessential E3 region. The adenoviral vector can be "multiply deficient," meaning that the adenoviral vector is deficient in one or more essential gene functions in each of two or more regions of the adenoviral genome. For example, the aforementioned E1-deficient or E1-, E3-deficient adenoviral vectors can be further deficient in at least one essential gene of the E4 region and/or at least one essential gene of the E2 region (e.g., the E2A region and/or E2B region). Adenoviral vectors deleted of the entire E4 region can elicit lower host immune responses. Examples of suitable adenoviral vectors include adenoviral vectors that lack (a) all or part of the E1 region and all or part of the E2 region, (b) all or part of the E1 region, all or part of the E2 region, and all or part of the E3 region, (c) all or part of the E1 region, all or part of the E2 region, all or part of the E3 region, and all or part of the E4 region, (d) at least part of the E1a region, at least part of the E1b region, at least part of the E2a region, and at least part of the E3 region, (e) at least part of the E1 region, at least part of the E3 region, and at least part of the E4 region, and (f) all essential adenoviral gene products (e.g., adenoviral amplicons comprising ITRs and the packaging signal only). As known to the skilled person, in case of deletions of essential regions from the adenovirus genome, the functions encoded by these regions have to be provided in trans, preferably by the producer cell, i.e. when parts or whole of E1, E2 and/or E4 regions are deleted from the adenovirus, these have to be present in the producer cell, for instance integrated in the genome, or in the form of so-called helper adenovirus or helper plasmids.

In further embodiments, the adenovirus of the invention lacks at least a portion of the E1-region, e.g. E1A and/or E1B coding sequences, and further comprises heterologous nucleic acid. Suitable heterologous nucleic acid is well known to the skilled person, and for instance may include transgene open reading frames, for instance open reading frames coding for polypeptides against which an immune response is desired when the rAd vector is used for vaccination purpose, e.g. transgenes suitable to generate an immune response against malaria (see e.g. WO 2004/055187), HIV, tuberculosis (see e.g. WO 2006/053871), certain viruses, etc, all well known to the skilled person. In fact, the nature of the heterologous nucleic acid is not critical to the instant invention, may be any heterologous nucleic acid, and hence needs no further elaboration here.

The person skilled in the art will be aware of the possibilities to propagate adenoviral vectors of different serotypes on specific host cells, using methods such as for instance disclosed in US patent 6,492,169 or in WO 03/104467, and references therein. For instance, for propagation of E1-deficient rAd26, specific producer cells that express E1B-55K of Ad26 can be constructed, for instance based on existing producer cells that express E1A and E1B of Ad5 such as PER.C6 or HEK293 cells (see, e.g. US 6,492,169), as is known to the skilled person. Alternatively and preferably, existing (Ad5-) complementing cell lines such as for instance PER.C6 or HEK293 can be used without modification of the cells for propagation of E1-deficient rAd26, by inclusion of the E4-orf6 coding sequence of Ad5 into the rAd26 vector, as extensively disclosed in for instance WO 03/104467.

Thus, propagation of adenoviral vectors of any serotype can be done on producer cells using means and methods well known to the person skilled in the art. Adenoviral vectors, methods for construction thereof and methods for propagating thereof, are well known in the art and are described in, for example, U.S. Pat. Nos. 5,559,099, 5,837,511, 5,846,782, 5,851,806, 5,994,106, 5,994,128, 5,965,541, 5,981,225, 6,040,174, 6,020,191, and 6,113,913, and Thomas Shenk, "Adenoviridae and their Replication", M. S. Horwitz, "Adenoviruses", Chapters 67 and 68, respectively, in Virology, B. N. Fields et al., eds., 3d ed., Raven Press, Ltd., New York (1996), and other references mentioned herein.

The construction of adenoviral vectors is well understood in the art and involves the use of standard molecular biological techniques, such as those described in, for example, Sambrook et al., Molecular Cloning, a Laboratory Manual, 2d ed., Cold Spring Harbor Press, Cold Spring Harbor, N.Y. (1989), Watson et al., Recombinant DNA, 2d ed., Scientific American Books (1992), and Ausubel et al., Current Protocols in Molecular Biology, Wiley Interscience Publishers, NY (1995), and other references mentioned herein.

Producer cells according to the invention are cultured to increase cell and virus numbers and/or virus titers. Culturing a cell is done to enable it to metabolize, and/or grow and/or divide and/or produce virus of interest according to the invention. This can be accomplished by methods as such well known to persons skilled in the art, and includes but is not limited to providing nutrients for the cell, for instance in the appropriate culture media. Different culture media can be used, and choosing the optimal culture medium for the cells and circumstances used is part of the routine tasks of the skilled person in this field. Suitable culture media for the purpose of the present invention are thus well known to the skilled person and can generally be obtained from commercial sources in large quantities, or custom-made according to standard protocols. Culturing can be done for instance in dishes, roller bottles or in bioreactors, using batch, fed-batch, continuous systems and the like. In order to achieve large scale (continuous) production of virus through cell culture it is preferred in the art to have cells capable of growing in suspension, and it is preferred to have cells capable of being cultured in the absence of animal- or human-derived serum or animal- or human-derived serum components. Suitable conditions for culturing cells are known (see e.g. Tissue Culture, Academic Press, Kruse and Paterson, editors (1973), and R.I. Freshney, Culture of animal cells: A manual of basic technique, fourth edition (Wiley-Liss Inc., 2000, ISBN 0-471-34889-9).

### Cell culture system and perfusion system

Bioreactors have been widely used for the large-scale production of biological products from suspension dependent animal cell cultures. According to the invention, the bioreactors used for adenovirus propagation can for instance be stirred tanks, disposable bioreactors, airlift reactors and the like. According to certain embodiments of the present invention the bioreactor used is a stirred tank.

According to certain embodiments of the invention, the bioreactor used has a working volume between about 2L and 2000L, between meaning herein to include the upper and lower limit values disclosed, i.e. 2L being the smallest working volume and 2000L being the largest working volume. Bioreactors used having a working volume of any individual value in between these values are meant to be included in the invention. The term 'about' for numerical values as used in the present disclosure means the value ± 10%. In certain preferred embodiments, the working volume is between 10L and 1000L, preferably between 20L and 800L, e.g. between 30L and 600L, e.g. between 50L and 500L, e.g. about 250 L or about 500L. An advantage of using bioreactors with a working volume according to the invention is that the use of very large volume bioreactors, i.e. those with a working volume of much more than 2000L, preferably 1000L, is avoided, and thus the huge capital and time investment in building such a very large bioreactor is not required. Further, the product, i.e. the rAd, is much more concentrated when use is made of the methods of the present invention, which saves time and costs in harvesting and/or further down stream processing of rAd from the bioreactors. The working volume is the effective culture volume in the bioreactor. The stirred tanks generally have a height-to-diameter ratio of 1:1 to 3:1. The culture is usually mixed with one or more agitators, based on bladed disks or marine propeller patterns. Agitator systems offering less shear forces than blades have been described. Agitation may be driven either directly or indirectly by magnetically coupled drives. Indirect drives reduce the risk of microbial contamination through seals on stirrer shafts. Instrumentation and controls of said bioreactors include (without limitation): agitation, temperature, dissolved oxygen, pH and biomass controls. The agitation, pH, temperature, dissolved oxygen concentration of the cell culture medium are in principle not critical and depend on the type of cell chosen. Preferably, the agitation, pH, temperature, dissolved oxygen concentration are chosen such that it is optimal for the growth of the cells. The person skilled in the art knows how to find the optimal agitation, pH, temperature, dissolved oxygen concentration for the culturing. Usually, the optimal agitation is between 50 and 300 rpm, e.g. 100-250 rpm, the optimal pH is between 6.7 and 7.7, the optimal temperature between 30 and 39°C, e.g. 34, 35, 36, 37 or 38°C.

Most large-scale suspension cultures are operated as batch or fed-batch processes because they are the most straightforward to operate and scale up. However, continuous processes based on perfusion principles are becoming more common. According to the present invention the producer cells are cultured in a perfusion system. Perfusion culturing of cells has its conventional meaning in the art, i.e. it means that during culturing cells are retained by a separation device in which there is an outflow of liquid having a lower cell density than prior to separation and in which there is an inflow of cell culture medium. The use of perfused culture is in response to the challenge of growing cells at high densities (e.g. 10-50 x 10⁶ viable cells/mL). In order to increase densities beyond 2-4 x 10⁶ viable cells/mL, the medium is constantly, or intermittently, replaced with a fresh supply in order to make up for nutritional deficiencies and to remove toxic products. Perfusion also allows for a far better control of the culture environment (pH, dO₂, nutrient levels, etc.). Perfusion of fresh medium through the culture can be achieved by retaining the cells with a variety of separation devices (e.g. fine mesh spin filter, hollow fiber or flat plate membrane filters, settling tubes). In preferred embodiments of the process of the present invention, the separation device is a filter module comprising hollow fibers.

With the term "hollow fiber" is meant a tubular membrane. The internal diameter of the tube is preferably between 0.3 and 6.0 mm, more preferably between 0.5 and 3.0 mm, most preferably between 0.5 and 2.0 mm. In certain embodiments, the mesh size (pore size) in the membrane is chosen such that the size of the pores in the mesh is close to the diameter of the cells, ensuring a high retention of cells while cell debris can pass the filter. In other embodiments, the mesh size is significantly smaller than the diameter of the cells. Preferably, the mesh size is between 0.1-30µm, e.g. between 0.1 and 3 µm, e.g. about 0.2 µm. Filter modules comprising hollow fibers are commercially available from for example General Electric (formerly Amersham). Significant amounts of adenovirus particles were not observed in the outflow culture medium during the process of the present invention, despite the virus particles being smaller than the applied mesh size.

Perfusion is used in order to maintain desired levels of certain metabolites and to remove and thereby reduce impurities in the culture medium. Perfusion rates can be measured in various manners, such as in terms of replacement volumes/unit time or in terms of levels of certain metabolites, which must be maintained, during periods of perfusion. It is typically the case that perfusion is not carried out at all times during culturing and is generally carried out only from time to time during culturing as desired. For example, perfusion is not typically initiated until after certain media components such as glucose begin to become exhausted and need to be replaced.

Several perfusion systems are known in the art and are in principle suitable for the methods of the present invention. With the term "perfusion system" is meant the combination of a bioreactor connected to a separation device. The separation device can either be incorporated in the bioreactor (e.g. fine mesh spin filter) or remain outside the bioreactor (e.g. hollow fiber). In both cases, as explained above, the separation device prevents washout of the cell mass from the reactor and enables medium refreshment.

The present inventors performed pilot experiments with several perfusion systems from which the Alternating Tangential Flow (ATF) perfusion system gave the best results. Therefore, in a preferred embodiment of the invention, the bioreactors are operated with (connected to) an ATF perfusion system (e.g. ATF System, Refine Technology, Co., East Hanover, NJ). The system consists of a diaphragm pump mounted to one end of a hollow fiber housing. The other end of the housing is attached to a joint assembly, which, in turn, is connected to a bioreactor through an available port. The diaphragm pump and control system serve to generate Alternating Tangential Flow through the hollow fibers. This means that there is one flow in the same direction as (i. e. tangential to) the membrane surfaces of the hollow fibers, which flow is going back and forth, and that there is another flow in a direction substantially perpendicular to said filter surface. Tangential flow can be achieved according to methods known to the person skilled in the art and as described in, for example, in US 6,544,424.

Operation of the ATF perfusion system has been described (Furey, 2002). ATF systems allow the cells to be cultured for a longer period of time and to reach high cell densities without having a blocked filter. Indeed, extremely high cell densities of over 100 x 10⁶ viable cells/mL can be obtained with the use of an ATF perfusion system, e.g. with PER.C6 cells (see e.g. Yallop et al). However, in earlier reports the PER.C6 cells in perfusion systems were used for a completely different purpose and not infected with adenovirus.

An additional advantage of the ATF system is that the system generates low shear stress. Energy is added to the surface of the liquid, generating a low shear laminar flow. This may be an advantage especially for the present invention, where cells are infected with adenovirus. During perfusion processes, post infection, with the ATF system no loss in cell density was found and no premature cell loss was observed, but rather even cell growth was observed. Since cells remain intact, optimal conditions are created for virus propagation.

The perfusion with the ATF system is therefore advantageous during the preculture phase (step a according to the present invention), since it allows obtaining very high cell densities, and the cells are in good condition for subsequent infection with adenovirus, possibly contributing to the high yields obtained. In order to reach said high cell densities, the culture medium is at least partially perfused during a portion of time during cell growth of the PER.C6 cells (step a). In certain aspects, perfusion is started once a cell density between about 2x10⁶ viable cells/mL and 8x10⁶ viable cells/mL is reached.

Further, the perfusion with the ATF system is advantageous after the infection stage (step c according to the present invention), since it allows obtaining very high adenovirus yields from the infected cells. In preferred aspects therefore, both the preculture stage and the post-infection stage of the processes of the invention employ an ATF perfusion system. The volume of culture medium used during ATF can be varied according to needs of the cells as can easily be established and adjusted by the skilled person, and typically varies between 0.5-5 vessel volumes/day (vol/d), e.g. between 1-3 vol/d, e.g. about 2 vol/d. In certain advantageous aspects, the refreshment rate is between about 1 and 2 vol/d, as the inventors have shown herein that this gives very good results in terms of yields and quality of the rAd26 obtained, while at the same time medium consumption and therefore costs associated therewith are still reasonable.

Finally the ATF perfusion system is a scalable system. Different size ATF units are available. Since airflow is used to drive the culture through the hollow fiber membrane, one can generate very rapid, low shear tangential flow rates enabling the technology to be used from R&D to production scale up to 1000 L (Furey, 2002). Possibly, further devolpments will allow even further upscaling of the ATF perfusion system.

In Yuk et al, rAd5 is produced using a tumor cell line, and therein the complete process is performed in a single bioreactor, which will take about 8-10 days in a production bioreactor. In certain embodiments of the present invention, two different bioreactors are used, one for the preculture (step a; preculture bioreactor), and one for the infection (step b) and post-infection culture (step c; production bioreactor) of the cells. An advantage of the use of two separate bioreactors for these steps is that only about 1.5-6, typically about 4-5 days of culturing in the production bioreactor are required, and therefore much more runs can be performed per year. Addition of a large amount of fresh culture medium during infection is further advantageous for reducing the volume of culture medium required during perfusion in the production bioreactor. Aternatively it is also possible to perform all steps a-c of the invention in a single bioreactor.

### Infection

In the methods of the invention, PER.C6 cells are infected with recombinant adenovirus 26. Typically, the virus will be exposed to the PER.C6 cell under optimal conditions, permitting uptake of the virus. The person skilled in the art knows how to find the optimal conditions, i.e. for agitation, pH, temperature, dissolved oxygen (dO₂ or DO), multiplicity of infection (MOI). Usually, the optimal agitation is between about 50 and 300 rpm, typically about 100-200, e.g. about 150, typical DO is 20-60%, e.g.40%, the optimal pH is between 6.7 and 7.7, the optimal temperature between 30 and 39°C, e.g. 34-37°C, and the optimal MOI between 5 and 1000, e.g. about 50-300. Typically, adenovirus 26 infects PER.C6 cells spontaneously, and bringing the PER.C6 cells into contact with rAd26 particles is sufficient for infection of the cells. Generally, an adenovirus seed stock is added to the culture to initiate infection, and subsequently the adenovirus propagates in the PER.C6 cells. This is all routine for the person skilled in the art.

In a certain aspect of the disclosure, perfusion is stopped prior to infection and is resumed after between 1 and 20 hours, e.g. 3-15 hours, e.g. 5 hours post infection. This delay should allow virus particles to enter the cells and prevent the virus particles from being flushed out of the system. Perfusion rates, post infection, are defined in terms of the glucose level that is maintained by means of the perfusion. For example, in the present disclosure the glucose concentration in the medium is usually maintained at a concentration of between about 2 mmol/L and 20 mmol/L, typically between about 5 and 10 mmol/L.

It was advantageously possible to infect a bioreactor with rAd26 at high cell densities, i.e. higher then 10x10⁶ viable cells/mL, with preservation of high virus productivity per cell. In certain embodiments, the specific productivity remains between about 0.5x10⁵ and 1.5x10⁵ VP/cell.

Moreover, in the present disclosure, the viability of the cell culture prior to infection remains higher then 75%. Meaning that at least 75% of the total amount of cells in the culture is viable at the moment of infection. In certain aspects the viability of the cell culture at infection is at least 80%, in further embodiments at least 85%. Viability can be measured using routine methods available to the skilled person, e.g. trypan blue exclusion, Casy cell count, and the like.

The cell density at infection is between about 10x10⁶ and 16x10⁶ viable cells/mL, e.g. between about 10x10⁶ and 15x10⁶ viable cells/mL, e.g. between about 10x10⁶ and 14x10⁶ viable cells/mL. These cell densities allow for high virus productivity with limited accumulation of cell debris and host cell DNA, which gives an advantage of these embodiments in down stream processing of the adenovirus harvest. Thus, the present invention provides an optimized process for rAd26 production, yielding high numbers of rAd26 particles of good quality, while at the same time providing a harvest material that is still manageable for down stream processing purposes.

Infections at these cell densities may produce even higher concentrations of recombinant adenovirus, in particular rAd26, and surpass the yields for rAd26 disclosed thus far. As shown for the first time in the present disclosure, in contrast to rAd5 infection at high cell densities (above 10x10⁶ viable cells/mL), infection with rAd26 at densities above 10x10⁶ viable cells/mL still increased the volumetric productivity of rAd26 with increasing cell densities up to at least 16x10⁶ viable cells/mL at infection, using producer cells in suspension with a perfusion system. In a preferred embodiment of the invention, a method is provided for producing at least 1x10¹² rAd26 virus particles (VP)/mL.

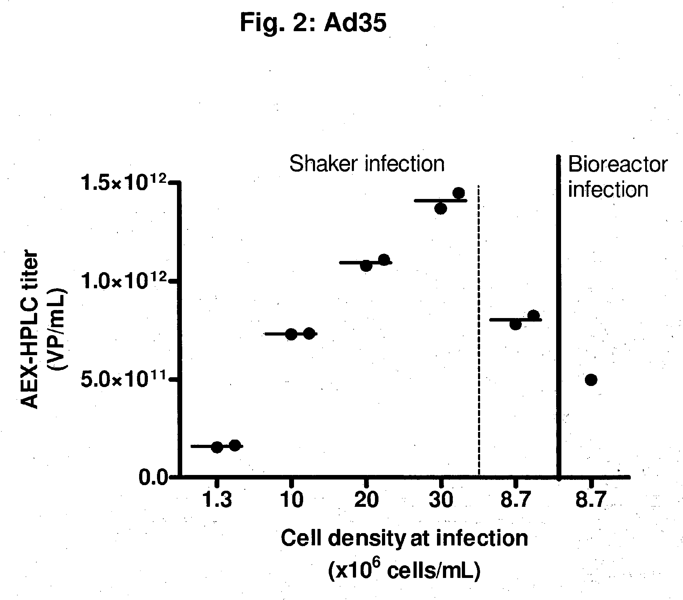

The processes of the current invention allow the recovery of rAd26 with a physical particle to infectious particle ratio of less than 30:1, which is an important parameter for adenovirus that is to be administered to humans. This can be measured as a virus particle (VP)/infectious unit (IU) ratio, for instance employing a QPA assay (Wang et al, 2005). A lower ratio is advantageous since less virus particles need to be administered to infect the same number of cells in such a case. Current FDA regulations require a VP/IU ratio of less than 30:1, and hence the processes of the invention described herein are suitable to prepare large numbers of rAd26 that fulfil this particular requirement. The authors of Yuk et al (2004) reported lower absolute numbers of virus particles than the numbers disclosed herein, and further the VP/IU ratio of the samples disclosed in Yuk et al (2004) are around 100 (Fig 2A/2B in Yuk et al, 2004). In contrast, we report higher absolute yields and moreover significantly better VP/IU ratios of below 20:1. In certain preferred embodiments therefore, the processes of the invention provide batches of rAd26 that have a VP/IU ratio of less than 20:1, e.g. between about 20:1 and about 5:1.

### Methods of cell harvest and lysis

After infection of an adenovirus, the virus replicates inside the cell and is thereby amplified. Adenovirus infection results finally in the lysis of the cells being infected. The lytic characteristics of adenovirus therefore permits two different modes of virus production. The first mode is harvesting virus prior to cell lysis, employing external factors to lyse the cells. The second mode is harvesting virus supernatant after (almost) complete cell lysis by the produced virus (see e.g. US patent 6,485,958, describing the harvesting of adenovirus without lysis of the host cells by an external factor). For the latter mode, longer incubation times are required in order to achieve complete cell lysis, and hence high yields of virus. Furthermore, the gradual spill of the host cell contents into the medium may be detrimental to the integrity and yield of the obtained viruses. Hence, it is preferred to employ external factors to actively lyse the cells for harvesting the adenovirus, according to the invention.

Methods that can be used for active cell lysis are known to the person skilled in the art, and have for instance been discussed in WO 98/22588, p. 28-35. Useful methods in this respect are for example, freeze-thaw, solid shear, hypertonic and/or hypotonic lysis, liquid shear, sonication, high pressure extrusion, detergent lysis, combinations of the above, and the like. In one embodiment of the invention, the cells are lysed using at least one detergent. Use of a detergent for lysis has the advantage that it is an easy method, and that it is easily scalable.

Detergents that can be used, and the way they are employed, are generally known to the person skilled in the art. Several examples are for instance discussed in WO 98/22588, p. 29-33. Detergents, as used herein, can include anionic, cationic, zwitterionic, and nonionic detergents. It is clear to the person skilled in the art that the concentration of the detergent may be varied, for instance within the range of about 0.1%-5% (w/w). In one embodiment, the detergent used is Triton X-100.

Nuclease may be employed to remove contaminating, i.e. mostly producer cell, nucleic acids. Exemplary nucleases suitable for use in the present invention include Benzonase^{®}, Pulmozyme^{®}, or any other DNase and/or RNase commonly used within the art. In preferred embodiments, the nuclease is Benzonase^{®}, which rapidly hydrolyzes nucleic acids by hydrolyzing internal phosphodiester bonds between specific nucleotides, thereby reducing the viscosity of the cell lysate. Benzonase^{®} can be commercially obtained from Merck KGaA (code W214950). The concentration in which the nuclease is employed is preferably within the range of 1-100 units/ml.

Methods for harvesting adenovirus from cultures of producer cells have been extensively disclosed in WO 2005/080556.

According to the , the time of harvest is between about 24 and 120 hours post infection, e.g. between about 48 and 96 hours post infection, e.g. 72 hours post infection.

### Methods of purification

In certain embodiments, the harvested adenovirus is further purified. Purification of the adenovirus can be performed in several steps comprising clarification, ultrafiltration, diafiltration or separation with chromatography as described in for instance WO 05/080556, incorporated by reference herein. Clarification may be done by a filtration step, removing cell debris and other impurities from the cell lysate. Ultrafiltration is used to concentrate the virus solution. Diafiltration, or buffer exchange, using ultrafilters is a way for removal and exchange of salts, sugars and the like. The person skilled in the art knows how to find the optimal conditions for each purification step. Also WO 98/22588, describes methods for the production and purification of adenoviral vectors. The methods comprise growing host cells, infecting the host cells with adenovirus, harvesting and lysing the host cells, concentrating the crude lysate, exchanging the buffer of the crude lysate, treating the lysate with nuclease, and further purifying the virus using chromatography.

Purification may for instance be achieved by density gradient centrifugation, as for instance discussed in WO 98/22588, p. 59-61.

Preferably however, purification employs at least one chromatography step, as for instance discussed in WO 98/22588, p. 61-70. Many processes have been described for the further purification of adenoviruses, wherein chromatography steps are included in the process. The person skilled in the art will be aware of these processes, and can vary the exact way of employing chromatographic steps to optimize the process.

It is for instance possible to purify adenoviruses by anion exchange chromatography steps, see for instance WO 05/080556. For adenovirus purification, it is preferred to use at least one anion exchange chromatography step. After the anion exchange chromatography step, the virus may be sufficiently pure. In certain embodiments however a size exclusion chromatography step is further performed to increase the robustness of the process. This step may be prior to or after the anion exchange chromatography step. Obviously, other purification steps may also be suitably combined with an anion exchange chromatography step.

The use of anion exchange chromatography for adenovirus purification has been extensively described, and this aspect is therefore well within the reach of the person skilled in the art. Many different chromatography matrices have been employed for purification of adenovirus and are suitable, and the person skilled in the art can easily find the optimal anion exchange material for purifying the virus, for instance guided by the following art.

US patent 5,837,520 (see also Huyghe et al., 1995, Human Gene Therapy 6: 1403-1416) describes a method of purifying adenovirus wherein the host cell lysate is treated with a nuclease, followed by anion exchange and metal ion affinity chromatography.

US patent 6,485,958 describes the use of strong anion exchange chromatography for purification of recombinant adenovirus.

Anion exchange chromatography has been employed with fluidized bed columns for the purification of adenovirus particles, see WO 00/50573.
Further, expanded bed anion exchange chromatography, and certain chromatographic resins for anion exchange chromatography for purification of adenovirus particles have been described in US patent 6,586,226.

In addition to anion exchange columns, anion exchange membrane chromatography products such as those produced by Pall (e.g. Mustang™ series) and Sartorius (e.g. Sartobind series) are suitable. For use of these filters and their advantages in adenovirus purification see for instance WO 03/078592 and WO 2005/080556.

US patent 6,537,793 describes the purification of adenoviral particles from host cells using ion-exchange chromatography, in particular teaching a preference for Q Sepharose XL types of chromatographic support for this purpose. In one embodiment of the present invention, an adenovirus is further purified using a Q Sepharose XL column.

The purification process may also suitably employ a size exclusion chromatography step.

International application WO 97/08298 describes the purification of adenoviruses using certain chromatographic matrices to prevent damage to the viruses, including anion exchange and size exclusion steps. US patent 6,261,823 describes a method for purifying adenovirus wherein the adenovirus preparation is subjected to anion exchange chromatography followed by size exclusion chromatography. In the size exclusion step, a group separation of viral particles from impurities of low molecular weight is achieved.

It is also possible to employ a hydroxyapatite medium for purifying adenovirus, see WO 02/44348.

A reversed-phase adsorption step might also be used, as for instance described in WO 03/097797, p. 26.

International application WO 97/08298 describes the purification of adenoviruses using certain chromatographic matrices to prevent damage to the viruses, including anion exchange and size exclusion steps.

Certain ultrafiltration methods are also very suitable for purification of adenovirus, as disclosed in WO 2006/108707. Such steps may be performed in addition to or instead of certain chromatographic purification steps.

Further advantageous purification methods for adenoviruses from high cell density cultures have been described by applicant in applications EP 09173090.3 and EP 09173119.0 as filed on 15 october 2009.

### Preparing a pharmaceutical preparation.

The purified adenovirus rAd26 may be formulated into a pharmaceutical composition. This can be done according to a variety of methods and using a variety of buffers all according to routine methods well known to the person skilled in the art. In general, it entails bringing the adenovirus particles in a pharmaceutically acceptable composition, comprising the adenovirus and at least a pharmaceutically acceptable excipient. Such a composition may be prepared under conditions known to the skilled person, and may be suitable for administration to humans.

For instance the adenovirus may be buffer exchanged during group separation to -and finally stored in- the buffer that is also used for the Adenovirus World Standard (Hoganson et al, Development of a stable adenoviral vector formulation, Bioprocessing March 2002, p. 43-48): 20 mM Tris pH 8, 25 mM NaCl, 2.5% glycerol.

Obviously, many other buffers can be used, and several examples of suitable formulations for the storage and pharmaceutical administration of purified (adeno)virus preparations can for instance be found in European patent no. 0853660, and in international patent applications WO 99/41416, WO 99/12568, WO 00/29024, WO 01/66137, WO 03/049763.

The adenovirus rAd26 vectors may be used as vaccines, and these are typically held in pharmaceutically acceptable carriers or excipients, and/or diluents. Pharmaceutically acceptable carriers or excipients and diluents are well known in the art and used extensively in a wide range of therapeutic products. Preferably, carriers are applied that work well in vaccines. More preferably, the vaccines further comprise an adjuvant. Adjuvants are known in the art to further increase the immune response to an applied antigenic determinant, and pharmaceutical compositions comprising adenovirus and an aluminium phosphate adjuvant are for instance disclosed in WO 2007/110409.

For administering to humans, pharmaceutical compositions comprising the rAd26 and a pharmaceutically acceptable carrier or excipient may be employed. In the present context, the term "Pharmaceutically acceptable" means that the carrier or excipient, at the dosages and concentrations employed, will not cause any unwanted or harmful effects in the subjects to which they are administered. Such pharmaceutically acceptable carriers and excipients are well known in the art (see Remington's Pharmaceutical Sciences, 18th edition, A. R. Gennaro, Ed., Mack Publishing Company [1990]; Pharmaceutical Formulation Development of Peptides and Proteins, S. Frokjaer and L. Hovgaard, Eds., Taylor & Francis [2000]; and Handbook of Pharmaceutical Excipients, 3rd edition, A. Kibbe, Ed., Pharmaceutical Press [2000]). The purified rAd26 preferably is formulated and administered as a sterile solution although it is within the scope of this invention to utilize lyophilized preparations. Sterile solutions are prepared by sterile filtration or by other methods known per se in the art. The solutions are then lyophilized or filled into pharmaceutical dosage containers. The pH of the solution generally is in the range of pH 3.0 to 9.5, e.g pH 5.0 to 7.5. The rAd26 typically is in a solution having a suitable pharmaceutically acceptable buffer, and the solution of rAd26 may also contain a salt. Optionally stabilizing agent may be present, such as albumin. Detergent may be added. RAd26 may be formulated into an injectable preparation. These formulations contain effective amounts of rAd26, are either sterile liquid solutions, liquid suspensions or lyophilized versions and optionally contain stabilizers or excipients.

The present invention disclosed methods to produce adenoviral vectors in rAd26, with very high yields, and as far as we are aware the yields obtained and disclosed herein have not been reported before. In the processes of the invention, bioreactors are used, and the bioreactor with the very high number of adenovirus particles per volume is a direct (intermediate) product of the . The invention therefore also provides use of a bioreactor in a method according to the invention, wherein the bioreactor has a working volume of between 2L and 2000L, preferably between 10L and 1000L, comprising: culture medium, PER.C6 cells, and at least 1x10¹² rAd26 virus particles (VP)/mL. The culture medium can be any culture medium suitable for propagation of the cells and infection for adenovirus, as described above. The aspects of the bioreactor volume, the PER.C6 cells and the number of rAd26 particles and VP/IU ratio are as described above for the methods of the invention. In preferred embodiments, the bioreactor is connected to an ATF perfusion system.

In yet another aspect, in the method of the invention at least 1x10¹² rAd26 virus particles (VP)/mL, the method comprising: a) culturing PER.C6 cells in suspension with a perfusion system; b) infecting said cells at a density of between 10x10⁶ viable cells/mL and 15x10⁶ viable cells/mL with rAd26; c) further culturing the infected cells with a perfusion system to propagate said rAd26, whereby the concentration of rAd26 virus particles reaches at least 1x10¹² VP/mL; and d) harvesting said rAd26. Prior to the instant disclosure, it was unknown if such high yields of rAd26 were feasible at all, let alone how to achieve such high yields. The instant invention discloses that these yields are possible according to methods disclosed herein. Preferably, the physical particle to infectious particle ratio of the harvested rAd26 is less than 30:1. Advantageous further embodiments are as described for the methods according to the invention as described supra.

The invention is further explained in the following examples. The examples do not limit the invention in any way. They merely serve to clarify the invention.

### EXAMPLES

### Example 1: Infection at high cell densities with an Ad5 vector. (Illustrative)

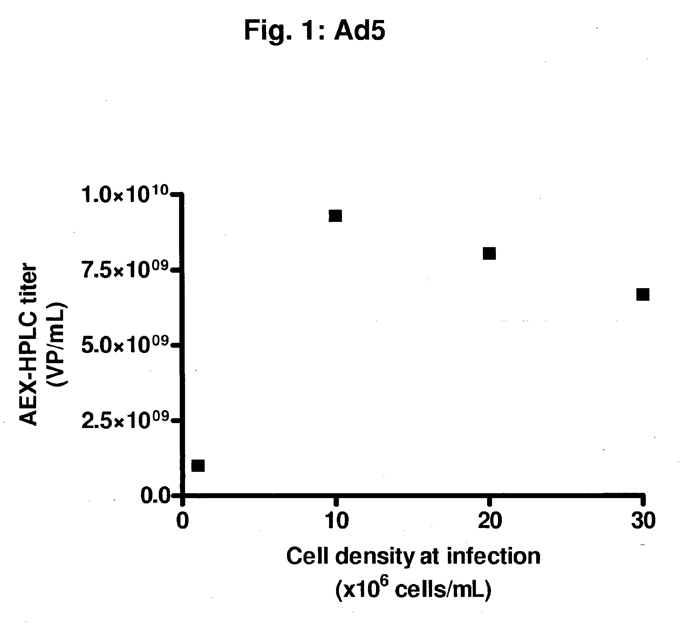

From a PER.C6^{®} working cell bank cells were thawed and propagated in serum free culture medium in a humidified incubator at 37°C and 10% CO₂. Subculture was performed every 3 to 4 days until sufficient cell density was reached to inoculate a 2L bioreactor at a volume of 1.5L and a cell density of 0.2 to 0.5x10⁶ viable cells/mL. Cells were propagated in the bioreactor at 37°C, DO of 40%, and a pH of 7.3. The ATF perfusion process was started at a cell density of 4.7x10⁶ total cells/mL. The ATF was from Refine Technology, Co., East Hanover, NJ. After 89 hours a cell density was reached of 12.4x10⁶ total cells/mL. At this moment a part of the cells were harvested and the cells were centrifuged for 5 minutes at 300g. The cell pellet was re-suspended to the following concentrations in fresh serum free medium:
- 1.3x10⁶ viable cells/mL, 30 mL/shaker, two 250mL shakers
- 10x10⁶ viable cells/mL, 30 mL/shaker, two 250mL shakers
- 20x10⁶ viable cells/mL, 30 mL/shaker, two 250mL shakers
- 30x10⁶ viable cells/mL, 30 mL/shaker, two 250mL shakers
The shakers were infected with Ad5.CS (a rand5 vector; Shott et al, 2008) at an MOI of 90 VP/cell and incubated at 36°C, 10% CO₂ and 100 rpm. Day 1 and 2 post infection medium refreshment was performed for the shakers infected at 10, 20, and 30x10⁶ viable cells/mL. This medium refreshment was performed by a centrifugation step for 5 minutes at 300g and re-suspending the cell pellet in 30mL fresh medium per shaker. Day 3 post infection the shakers were harvested and sampled for AEX-HPLC analysis. Cell lysis of the harvest was performed by mixing 1 mL sample volume of each shaker with 100 µL 10% Triton X-100 and incubation at 37°C for 30 minutes. After incubation the samples were mixed with 2.42 µL benzonase/MgCl₂ followed by a subsequent incubation step of 30 minutes at 37°C. Finally 100 µL 50% sucrose was added to samples. After a centrifugation step of 5 minutes at 2500g, the samples were stored below -65°C until analysis by AEX-HPLC was performed to determine the number of virus particles produced (VP/mL). The results are presented in Fig. 1.

The volumetric yield of an infection at a cell density of 10x10⁶ viable cells/mL was 10 fold higher then at 1x10⁶ viable cells/mL. This was somewhat unexpected given the cell density effect reported in earlier reports at much lower densities (i.e., at about 0.5-3x10⁶ cells/mL, e.g. Maranga et al., 2005; Kamen et al., 2004; Altaras et al., 2005). However, past the 10x10⁶ cells/mL, a cell density effect was observed and volumetric yields decreased. Thus, with recombinant Ad5 a cell density effect is seen in the perfusion system.

### Example 2: Infection with rAd35 at low cell densities (1-1.6x10⁶ viable cells/mL). (Illustrative)

In example 1, rand5 was used. However, different adenovirus serotypes are known and have been described for different purposes. These serotypes may have different properties, and hence processes useful for one serotype are not always necessarily suitable for another serotype. This may especially be relevant in industrial scale processes, where seemingly small differences may be economically of great importance. One particularly advantageous serotype for use in for instance vaccines is Ad35, and in the following examples we test the feasibility to improve yields of rAd35 to obtain large quantities thereof. This example shows infection with a rAd35 vector at low cell densities, as a comparison to the following examples where cells are infected at higher cell densities.

From a PER.C6^{®} working cell bank, cells were thawed and propagated in serum free culture medium in a humidified incubator at 37°C and 10% CO₂. Subculture was performed every 3 to 4 days until sufficient cell density was reached to inoculate 10L bioreactors at a volume of 5L and a cell density of 0.2 to 0.35x10⁶ viable cells/mL. Cells were propagated in the bioreactor at 37°C, DO of 40%, and a pH of 7.3. Four days after inoculation (when a cell density was reached between 2 to 3.5x10⁶ viable cells/mL) the cell suspension was diluted with 5L fresh medium and subsequently infected with rAd35.TB-S (a rAd35 vector; Radosevic et al, 2007) at an MOI of 70 VP/cell. Virus propagation was performed at 36°C, pH 7.3 and DO 40%. Three days after infection the bioreactors were sampled for cell count and virus production determination. To release the virus, 1 mL sample of each bioreactor was mixed with 100 µL 10% Triton X-100 and incubated at 37°C for 30 minutes. After incubation the samples were mixed with 2.42 µL benzonase/MgCl₂ followed by a subsequent incubation step of 30 minutes at 37°C. Finally 100 µL 50% sucrose was added to the samples. After a centrifugation step of 5 minutes at 2500g, the samples were stored at a temperature below -65°C until analysis by AEX-HPLC. A total of ten bioreactor runs were performed and analyzed according to above described process, and these runs gave consistent results (not shown). The average virus particle production was 2.3x10¹¹ VP/mL.

For a yearly demand of about 1.5x10¹⁹ VP, with such a yield about 65000 L would have to be processed. This would require large facilities and therefore large upfront investment during vaccine development.

### Example 3: Feasibility study of an infection process with rAd35 at high cell densities (>10x10⁶ viable cells/mL). (Illustrative)

From a PER.C6^{®} working cell bank cells were thawed and propagated in serum free culture medium in a humidified incubator at 37°C and 10% CO₂. Subculture was performed every 3 to 4 days until sufficient cell density was reached to inoculate a 2L bioreactor at a volume of 1.5L and a cell density of 0.2 to 0.5x10⁶ viable cells/mL. Cells were propagated in the bioreactor at 37°C, DO of 40%, and a pH of 7.3. Medium perfusion was started at a cell density of 6.8x10⁶ total cells/mL, using an ATF system. After 70 hours a cell density was reached of 36.8x10⁶ total cells/mL. At this moment the following infections were performed:
- Infection in shakers at cell densities of:
   o 1.3x10⁶ viable cells/mL, 30 mL/shaker, two 250mL shakers
   o 10x10⁶ viable cells/mL, 30 mL/shaker, two 250mL shakers
   o 20x10⁶ viable cells/mL, 30 mL/shaker, two 250mL shakers
   o 30x10⁶ viable cells/mL, 30 mL/shaker, two 250mL shakers
- Infection at 2L bioreactor scale at 8.7x10⁶ total cells/mL (84% viability)
   o One hour after infection of the bioreactor a sample was withdrawn from the bioreactor and transferred to two 250mL shakers, 30 mL/shaker
For the infection process in 250 mL shakers a part of the cell suspension from the 2L bioreactor was harvested and this suspension was centrifuged for 5 minutes at 300g. The cell pellet was re-suspended to the above-mentioned concentrations in fresh serum free medium. The shakers were infected with Ad35.TB-S at an MOI of 70 VP/cell and incubated at 36°C, 10% CO₂ and 100 rpm. Day 1 and 2 post infection medium refreshment was performed for the shakers infected at 10, 20, and 30x10⁶ viable cells/mL. This medium refreshment was performed by a centrifugation step for 5 minutes at 300g and re-suspending the cell pellet in 30mL fresh medium per shaker. Day 3 post infection the shakers were harvested and sampled for AEX-HPLC analysis. Cell lyses of the harvest was performed by mixing 1 mL sample volume of each shaker with 100 µL 10% Triton X-100 and incubation at 37°C for 30 minutes. After incubation the samples were mixed with 2.42 µL benzonase/MgCl₂ followed by a subsequent incubation step of 30 minutes at 37°C. Finally 100 µL 50% sucrose was added to samples. After a centrifugation step of 5 minutes at 2500g, the samples were stored below -65°C until analysis by AEX-HPLC was performed.

The remaining cells in the 2L bioreactor were diluted with fresh serum free medium to a cell concentration of 8.7x10⁶ total cells/mL (84% viability). The bioreactor was infected with Ad35.TB-S at an MOI of 70 VP/cell and incubated at 36°C, pH 7.3 and DO of 40%. The ATF system was started 15 hours after infection at a medium refreshment rate of 1 bioreactor volume per day. Day 1, 2, 3, and 4 post infection the bioreactor was sampled for cell count (CASY cell counter) and virus production determination by AEX-HPLC. Sample preparation was performed as described above. The samples were stored below -65°C until analysis by AEX-HPLC was performed.

Approximately one hour after infection of the bioreactor a sample of at least 60 mL was taken from the 2L bioreactor and two infections (in 250mL shakers) were started at a volume of 30mL per shaker. Day 1 and 2 post infection medium refreshment was performed to mimic the perfusion system. This medium refreshment was performed by a centrifugation step for 5 minutes at 300g and re-suspending the cell pellet in 30mL fresh medium per shaker. Day 3 post infection the shakers were harvested and sampled for AEX-HPLC analysis. Sample preparation was performed as described above. The samples were stored below -65°C until analysis by AEX-HPLC was performed.

Results are presented in Fig. 2. Results show that infection between 1.3x10⁶ viable cells/mL and 30x10⁶ viable cells/mL is possible. In contrast to the results with rAd5, the total yields of rAd35 increased with increasing cell density at infection even above the 10x10⁶ viable cells/mL samples. At 30x10⁶ viable cells/mL a volumetric yield of 1.4x10¹² VP/mL was reached. The results clearly indicate that infections with Ad35.TB-S at high cell densities, i.e. 10x10⁶ viable cells/mL or higher, are possible. Even at 30x10⁶ viable cells/mL, infections gave high volumetric yields.
It is noted that a decrease is seen in unit productivity from 120.000 VP/cell at 1.3x10⁶ cells to 47.000 VP/cell at 30x10⁶ viable cells/mL. The shakers started from a cell suspension, which was infected in the bioreactor, show a harvest yield of 8.0x10¹¹ VP/mL and a unit productivity of 92.000 VP/cell. The results in the 2L bioreactor are somewhat lower: a harvest yield of 5x10¹¹ VP/mL was obtained, which is a unit productivity of 57.000 VP/cell.

### Example 4: Bioreactor experiments of infection at high cell densities with a rAd35 vector. (Illustrative)

1) From a PER.C6^{®} working cell bank cells were thawed and propagated in serum free culture medium in a humidified incubator at 37°C and 10% CO₂. Subculture was performed every 3 to 4 days until sufficient cell density was reached to inoculate a 2L bioreactor at a cell density of 0.59x10⁶ viable cells/mL. Cells were propagated in the 2L bioreactor at 37°C, DO of 40%, and a pH of 7.3. When a cell density of approximately 2.9x10⁶ total cells/mL was reached (day 4 post inoculation) an ATF system was started. After 118 hours of perfusion a cell density of 29x10⁶ total cells/mL was reached. At this moment a part of the cell suspension was harvested and the remaining cells were diluted with fresh medium in the 2L bioreactor to a cell density of 16.4x10⁶ total cells/mL (82% viability, hence 13.4x10⁶ viable cells/mL). Subsequently the 2L bioreactor was infected with Ad35.TB-S at an MOI of 70 VP/cell and incubated at 36°C, pH 7.3 and DO of 40%. The ATF system was started 15 hours post infection at a medium refreshment rate of 2 vessel volume per day. On day 1, 2, and 3 post infection the 2L bioreactor was sampled for cell count and virus production by AEX-HPLC. To release the virus 1 mL sample was mixed with 100 µL 10% Triton X-100 and incubated at 37°C for 30 minutes. After incubation the sample was mixed with 2.42 µL benzonase/MgCl₂ followed by a subsequent incubation step of 30 minutes at 37°C. Finally 100 µL 50% sucrose was added to the samples. After a centrifugation step of 5 minutes at 2500g the samples were stored at a temperature below -65°C until analysis by AEX-HPLC. The results are presented in Table 2.

The results demonstrated that infections at cell densities above 10x10⁶ viable cells/mL are feasible in bioreactors coupled to a perfusion system and that it is possible to increase the volumetric yield almost 10 times compared to a batch process (example 2). No premature cell loss of the infected culture was observed, indicating that the ATF process is an appropriate system for culturing infected cells.

**Table 2: Results example 4 (1).**

| Day post infection | Cell count (x10⁶ total cells/mL) | AEX-HPLC (VP/mL) | QPA (IU/mL) | AEX/QPA (VP/IU) |
|---|---|---|---|---|
| 0 | 16.4 | NA | NA | NA |
| 1 | 21.4 | Below LOQ | Below LOQ | NA |
| 2 | 29.18 | 1.34 x10¹² | 2.23 x10¹¹ | 6.0 |
| 3 | 31.50 | 2.26 x10¹² | 3.16 x10¹¹ | 7.2 |

An FDA requirement for rAd batches is a ratio of VP/IU < 30. QPA (Q-PCR based potency assay; Wang et al, 2005) analysis showed that all samples met this requirement. In contrast, the samples disclosed in Yuk et al (2004) have a VP/IU ratio of around 100 (Fig 2A/2B therein). The physical particles to infectious particles ratio is a relevant parameter for adenoviruses, and a lower ratio is preferred for rAd, batches. The batches prepared in this example consistently have such a low ratio of less then 10:1.
For a yearly demand of about 1.5x10¹⁹ VP and with a yield of about 2x10¹² VP/ml, less than 7500 L harvest have to be processed. These volumes can be processed in facilities of 1000L or less, and would thus reduce the upfront cost commitment during vaccine development.

### 2) Further experiments in 2L bioreactor

From a PER.C6^{®} working cell bank cells were thawed and propagated in serum free culture medium in a humidified incubator at 37°C and 10% CO₂. Subculture was performed every 3 to 4 days until sufficient cell density was reached to inoculate a 2L bioreactor at a cell density of 0.44x10⁶ total cells/mL. Cells were propagated in the 2L bioreactor at 37°C, DO of 40%, and a pH of 7.3. The ATF system was started 4 days post inoculation at a cell density of approximately 2.72x10⁶ total cells/mL. After 144 hours of perfusion a cell density of 30.5x10⁶ total cells/mL was reached. At this moment a part of the cell suspension was harvested and the remaining cells were diluted with fresh medium in the 2L bioreactor to a cell density of 16.2x10⁶ total cells/mL (81% viability, hence 13.1x10⁶ viable cells/mL). Subsequently the 2L bioreactor was infected with Ad35.TB-S at an MOI of 70 VP/cell and incubated at 36°C, pH 7.3 and DO of 40%. The ATF system was started 5 hours post infection at a medium refreshment rate of 2 vessel volume per day. On day 2, 3, and 4 post infection the 2L bioreactor was sampled for cell count and virus production by AEX-HPLC. To release the virus 1 mL sample was mixed with 100 µL 10% Triton X-100 and incubated at 37°C for 30 minutes. After incubation the sample was mixed with 2.42 µL benzonase/MgCl₂ followed by a subsequent incubation step of 30 minutes at 37°C. Finally 100 µL 50% sucrose was added to the samples. After a centrifugation step of 5 minutes at 2500g the samples were stored at a temperature below -65°C until analysis by AEX-HPLC. The results are presented in Table 3.

**Table 3: Results example 4 (2).**

| Day post infection | Cell count (x10⁶ total cells/mL) | AEX-HPLC (VP/mL) | QPA (IU/mL) | AEX/QPA (VP/IU) |
|---|---|---|---|---|
| 0 | 1.6.19 | NA | NA | NA |
| 1 | 20.40 | NA | NA | NA |
| 2 | 24.14 | 1.42 x10¹² | 1.77 x10¹¹ | 8.0 |
| 3 | 24.60 | 2.20 x10¹² | 1.82 x10¹¹ | 12.1 |
| 4 | 16.26 | 1.90 x10¹² | 1.51 x10¹¹ | 12.5 |

The results again demonstrated that infections at cell densities above 10x10⁶ viable cells/mL are feasible in bioreactors coupled to a perfusion system and that it is possible to increase the volumetric yield almost 10 times compared to a batch process (example 2). Furthermore this example demonstrates that the perfusion rate after infection can be limited to 2 vessel volumes per day without compromising the virus production.

For a yearly demand of about 1.5x10¹⁹ VP and with a yield of about 2x10¹² VP/ml, less than 7500 L harvest have to be processed. These volumes can be processed in facilities of 1000L or less, and would thus reduce the upfront cost commitment during vaccine development.

### 3) Further experiments in 50L bioreactor

From a PER.C6^{®} working cell bank cells were thawed and propagated in serum free culture medium in a humidified incubator at 37°C and 10% CO₂. Subculture was performed every 3 to 4 days until sufficient cell density was reached to inoculate a 10L bioreactor at a cell density of 0.52x10⁶ total cells/mL. Cells were propagated in the 10L bioreactor at 37°C, DO of 40%, and a pH of 7.3. The ATF system was started when a cell density of approximately 5.3x10⁶ total cells/mL was reached (4 days post inoculation). After 169 hours of perfusion a cell density of 77x10⁶ total cells/mL was reached. At this moment the 10L cell suspension was diluted with fresh medium in a 50L bioreactor to a cell density of 15.5x10⁶ total cells/mL (81% viability, hence 12.6x10⁶ viable cells/mL). Subsequently the 50L bioreactor was infected with Ad35.TB-S at an MOI of 70 VP/cell and incubated at 36°C, pH 7.3 and DO of 40%. The ATF system was started 5 hours post infection at a medium refreshment rate of 2 vessel volume per day. On day 2 and 3 post infection the 50L bioreactor was sampled for cell count and virus production by AEX-HPLC. To release the virus 1 mL sample was mixed with 100 µL 10% Triton X-100 and incubated at 37°C for 30 minutes. After incubation the sample was mixed with 2.42 µL benzonase/MgCl₂ followed by a subsequent incubation step of 30 minutes at 37°C. Finally 100 µL 50% sucrose was added to the samples. After a centrifugation step of 5 minutes at 2500g the samples were stored at a temperature below -65°C until analysis by AEX-HPLC. The results are presented in Table 4.

**Table 4: Results example 4 (3).**

| Day post infection | Cell count (x10⁶ total cells/mL) | AEX-HPLC (VP/mL) | QPA (IU/mL) | AEX/QPA (VP/IU) |
|---|---|---|---|---|
| 0 | 15.5 | NA | NA | NA |
| 2 | 21.4 | 1.67 x10¹² | 1.15 x10¹¹ | 14.6 |
| 3 | 23.5 | 1.84 x10¹² | 1.99 x10¹¹ | 9.2 |

The results demonstrated that infections at cell densities above 10x10⁶ viable cells/mL were feasible in 50L bioreactors coupled to a perfusion system and that it was possible at 50L scale to increase the volumetric yield almost 10 times compared to a batch process (example 2). It was shown herewith that the developed process could be scaled-up. The harvest volumes that must be processed per year in order to fulfil the yearly virus demand, can be produced with the current process. For a yearly demand of about 1.5x10¹⁹ VP and with a yield of about 2x10¹² VP/ml, less than 7500 L harvest have to be processed. These volumes can be processed in facilities of 1000L or less, and would thus reduce the upfront cost commitment during vaccine development.

### Example 5: Infection with rAd26 at low cell densities (1-1.6x10⁶ viable cells/mL).

This example shows infection with a rAd26 vector comprising a CS malaria transgene (rAd26.CS) at low cell densities, as a comparison to later examples where cells are infected at higher cell densities.

From a PER.C6^{®} working cell bank, cells were thawed and propagated in serum free culture medium in a humidified incubator at 37°C and 10% CO₂. Subculture was performed every 3 to 4 days until sufficient cell density was reached to inoculate 10L bioreactors at a volume of 5L and a cell density of 0.25 to 0.35x10⁶ viable cells/mL. Cells were propagated in the bioreactor at 37°C, DO of 40%, and a pH of 7.3. Three days after inoculation (when a cell density was reached between 1 to 2.1x10⁶ viable cells/mL) the cell suspension was diluted with 5L fresh medium and subsequently infected with a rAd26.CS vector at an MOI of 70 VP/cell. Virus propagation was performed at 36°C, pH 7.3 and DO 40%. Three days after infection the bioreactors were sampled for cell count and virus production determination. To release the virus, 1 mL sample of each bioreactor was mixed with 100 µL 10% Triton X-100 and incubated at 37°C for 30 minutes. After incubation the samples were mixed with 2.42 µL benzonase/MgCl₂ followed by a subsequent incubation step of 30 minutes at 37°C. Finally 100 µL 50% sucrose was added to the samples. After a centrifugation step of 5 minutes at 2500g, the samples were stored at a temperature below -65°C until analysis by AEX-HPLC. A total of 5 bioreactor runs were performed and analyzed according to above described process, and these runs gave consistent results (not shown). The average virus particle production was 7.6x10¹⁰ VP/mL.

For a yearly demand of about 1.5x10¹⁹ VP, with such a yield about 197000 L would have to be processed annualy. This would require large facilities and therefore large upfront investment during vaccine development.

### Example 6: Feasibility of infection at high cell densities with rAd26 vector.

The feasibility of infection at high cell densities with Ad35 was previously assessed and it was shown that the production of Ad35 could be upscaled. Since substantial differences exists between the adeno serotypes it was herein tested whether the production of Ad26 by infecting high cell density cultures was feasible.

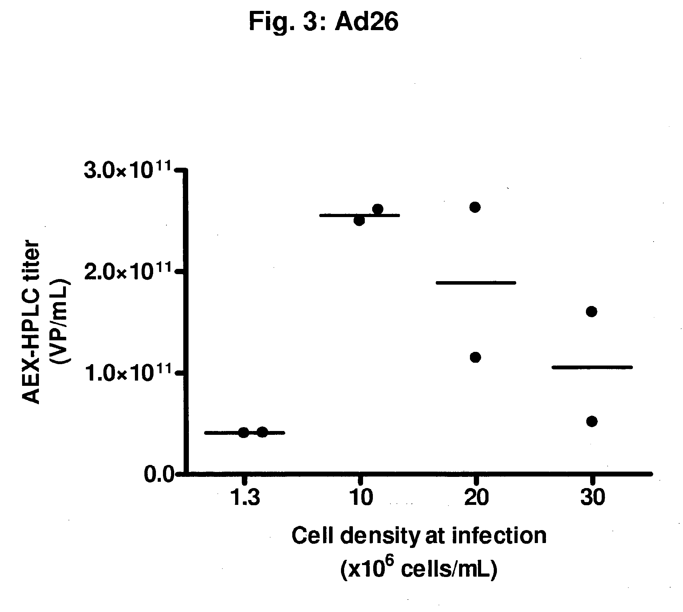

From a PER.C6^{®} working cell bank cells were thawed and propagated in serum free culture medium in a humidified incubator at 37°C and 10% CO₂. Subculture was performed every 3 to 4 days until sufficient cell density was reached to inoculate a 2L bioreactor at a volume of 1.5L and a cell density of 0.5x10⁶ viable cells/mL. Cells were propagated in the bioreactor at 37°C, DO of 40%, and a pH of 7.3. The ATF perfusion process was started at a cell density of 3.6x10⁶ total cells/mL. After 120 hours a cell density was reached of 21.6x10⁶ total cells/mL. At this moment a part of the cells were harvested. The cells were centrifuged for 5 minutes at 300g and the cell pellet was re-suspended to the following concentrations in fresh serum free medium:
- 1.3x10⁶ viable cells/mL, 30 mL/shaker, two 250mL shakers
- 10x10⁶ viable cells/mL, 30 mL/shaker, two 250mL shakers
- 20x10⁶ viable cells/mL, 30 mL/shaker, two 250mL shakers
- 30x10⁶ viable cells/mL, 30 mL/shaker, two 250mL shakers
The shakers were infected with rAd26.CS at an MOI of 70 VP/cell and incubated at 36°C, 10% CO₂ and 100 rpm. Day 1 and 2 post infection medium refreshment was performed for the shakers infected at 10, 20, and 30x10⁶ viable cells/mL. This medium refreshment was performed by a centrifugation step for 5 minutes at 300g and re-suspending the cell pellet in 30mL fresh medium per shaker. Day 3 post infection the shakers were harvested and sampled for AEX-HPLC analysis. Cell lysis of the harvest was performed by mixing 1 mL sample volume of each shaker with 100 µL 10% Triton X-100 and incubation at 37°C for 30 minutes. After incubation the samples were mixed with 2.42 µL benzonase/MgCl₂ followed by a subsequent incubation step of 30 minutes at 37°C. Finally 100 µL 50% sucrose was added to samples. After a centrifugation step of 5 minutes at 2500g, the samples were stored below -65°C until analysis by AEX-HPLC was performed. The results are presented in Fig. 3 and show that infecting cells with Ad26 at a cell density up to 30x10⁶ viable cells/mL was feasible. The rAd26 volumetric yields were higher than the yields obtained with e.g. Ad5.

In contrast to the results with rAd35, a cell density effect was observed at cell densities higher than 10x10⁶ viable cells/mL. Indeed, the rAd26 yields increased when increasing the cell densities at infection above 10x10⁶ viable cells/mL and the yields decreased in the range of 20x10⁶ - 30x10⁶ viable cells/mL.

Based on the observed cell density effect, an optimal cell density at infection could be determined for Ad26. Said optimum lies within the range of 10-16 x10⁶ viable cells/ml. The volumetric yields of rAd26 obtained by infecting cells in said range were substantially higher as compared to volumetric yields obtained by infecting cells at low cell densities (e.g. in a batch process). Indeed, the yield obtained at low cell density in example 5 was equal to 7.6x10¹⁰ VP/ml, while at high cell densities at infection, the yields reached up to 1-2x10¹² VP/ml (see example 7). Infecting at said range allows for having a highly productive process that can be used at large production scales.

### Example 7: Infection at high cell densities with an rAd26 vector in a bioreactor.

From a PER.C6^{®} working cell bank cells were thawed and propagated in serum free culture medium in a humidified incubator at 37°C and 10% CO₂. Subculture was performed every 3 to 4 days until sufficient cell density was reached to inoculate a 2L bioreactor at a cell density of 0.6x10⁶ total cells/mL. Cells were propagated in the 2L bioreactor at 37°C, DO of 40%, and a pH of 7.3. The ATF system was started 2 days post inoculation at a cell density of approximately 2x10⁶ total cells/mL. After 7 days of perfusion a cell density of 19.1x10⁶ total cells/mL was reached. At this moment a part of the cell suspension was harvested and the remaining cells were diluted with fresh medium in the 2L bioreactor to a cell density of 16.5x10⁶ total cells/mL (80% viability, hence 13.1x10⁶ viable cells/mL). Subsequently the 2L bioreactor was infected with rAd26.CS at an MOI of 70 VP/cell and incubated at 36°C, pH 7.3 and DO of 40%. The ATF system was started 5 hours post infection at a medium refreshment rate of 2 vessel volumes per day. On day 3, 4, 5 and 6 post infection the 2L bioreactor was sampled for cell count and virus production by AEX-HPLC and QPA. To release the virus 1 mL sample was mixed with 100 µL 10% Triton X-100 and incubated at 37°C for 30 minutes. After incubation the sample was mixed with 2.42 µL benzonase/MgCl₂ followed by a subsequent incubation step of 30 minutes at 37°C. Finally 100 µL 50% sucrose was added to the samples. After a centrifugation step of 5 minutes at 2500g the samples were stored at a temperature below -65°C until analysis by AEX-HPLC and QPA. The results are presented in Table 5.

**Table 5: Results example 7.**

| Day post infection | Cell count (x10⁶ total cells/mL) | AEX-HPLC (VP/mL) | QPA (IU/mL) | AEX/QPA (VP/IU) |
|---|---|---|---|---|
| 0 | 16.5 | NA | NA | NA |
| 3 | 31.2 | 4.4 x10¹¹ | 5.37 x10¹⁰ | 8.2 |
| 4 | 33.8 | 1.3 x10¹² | 1.38 x10¹¹ | 9.4 |
| 5 | 28.1 | 1.42 x10¹² | 1.54 x10¹¹ | 9.2 |
| 6 | 20.3 | 1.64 x10¹² | 9.94 x10¹⁰ | 16.5 |

These results for the first time demonstrate that infections of rAd26 at cell densities between above 10x10⁶ and 16x10⁶ viable cells/mL are feasible in bioreactors coupled to a perfusion system and that it is possible to increase the volumetric yield more than 20 times compared to a batch process (as shown in example 5).

For a yearly demand of about 1.5x10¹⁹ VP and with a yield of about 2x10¹² VP/ml, less than 7500 L harvest have to be processed. These volumes can be processed in facilities of 1000L or less, and would thus reduce the upfront cost commitment during vaccine development.

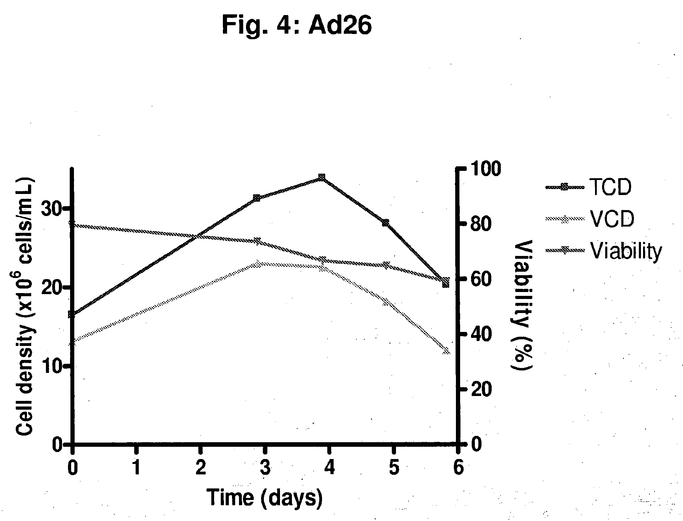

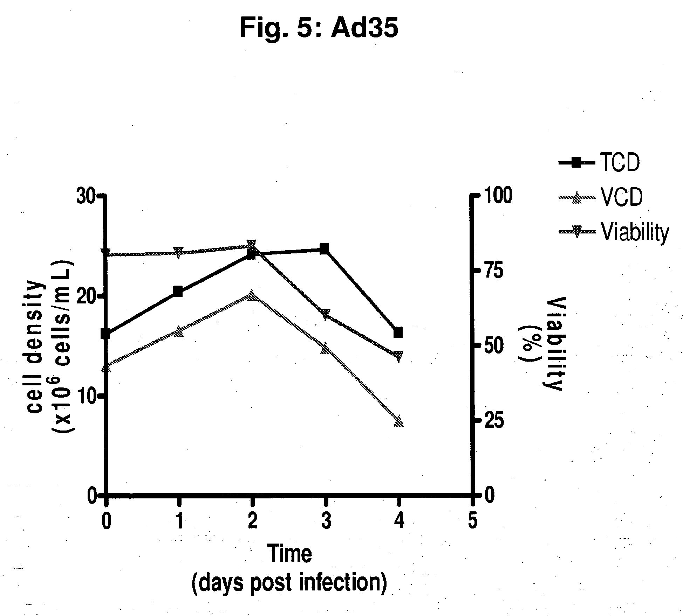

When comparing cell growth post infection , it was observed that after infection with Ad26, cells tend to grow further, while after infection with Ad35, cells stopped growing. Indeed, it was shown (Fig. 4) that cells which were infected with Ad26 at a density of 12x10⁶ vc/ml grew further to a maximum of 22x10⁶ vc/ml after 3 days. After infection with Ad35, cells that were infected at a density of 12x10⁶ vc/ml grew further to a maximum at day 2 and dropped to a density of 14x10⁶ vc/ml after 3 days (Fig. 5). Thus, Ad26 propagates differently then Ad35. Together with the cell density effect described hereabove, this shows that there are clear differences between propagation of recombinant adenoviruses of different serotypes (Ad5, Ad35 and Ad26) in cells, which differences have impact on industrial scale processes for generating pharmaceutical materials from these serotypes.

### References

Altaras NE, Aunins JG, Evans RK, Kamen A, Konz JO, Wolf JJ. Production and formulation of adenovirus vectors. Adv. Biochem. Engin/Biotechnol. Vol. 99, 2005
Cortin V, Thibault J, Jacob D, Garnier A. High-Titer adenovirus vector production in 293S Cell Perfusion culture. Biotechnol. Prog. Vol. 20, 2004
Estape D, Wilde F. Comparison of campaign vs concurrent large-scale cell culture facilities. Pharmaceutical Engineering. Vol. 26, No. 5, September/October 2006.
Furey J. Scale-up of a cell culture perfusion process - A low-shear filtration system that inhibits filter-membrane fouling. Genetic Engineering News. Vol. 22, No. 7, April 2002.
Henry O, Dormond E, Perrier M, Kamen A. Insights into adenoviral vector production kinetics in acoustic filter-based perfusion cultures. Biotechnology and bioengineering, Vol. 86, No. 7, June 2004.
Hodge G. Disposable bioprocessing: State of the Industry, Economics and a novel manufacturing platform case study (Presentation). NC Bio. Ctr. BPD Conference, 18 November 2004.
Kamen A, Henry O. Development and optimization of an adenovirus production process. The journal of gene medicin Vol. 6, 2004
Maranga L, Aunins JG, Zhou W. Characterization of changes in PER.C6 cellular metabolism during growth and propagation of a replication-deficient adenovirus vector. Biotechnology and Bioengineering, Vol. 90, No. 5, June 2005.
Radosevic K, Wieland CW, Rodriguez A, Weverling GJ, et al. Protective immune responses to a recombinant adenovirus type 35 tuberculosis vaccine in two mouse strains: CD4 and CD8 T-cell epitope mapping and role of gamma interferon. Infect. Immun. 75: 4105-4115 (2007).
Shott JP, McGrath SM, Pau MG, Custers JH, et al. Adenovirus 5 and 35 vectors expressing Plasmodium falciparum circumsporozoite surface protein elicit potent antigen-specific cellular IFN-gamma and antibody responses in mice. Vaccine 26: 2818-2823 (2008).
United Nation web site: http://esa.un.org/unpp/index.asp?panel=2 (March 2008)
Wang F, Puddy AC, Mathis BC, Montalvo AG, et al. Using QPCR to assign infectious potencies to adenovirus based vaccines and vectors for gene therapy: toward a universal method for the facile quantitation of virus and vector potency. Vaccine 23: 4500-4508 (2005).
Xie L, Metallo C, Warren J, Pilbrough W, Peltier J, Zhong T, Pikus L, Yancy A, Leung J, Aunins JG, Zhou W. Large-Scale propagation of a replication-defective adeno vector in stirred-tank bioreactor PER.C6 cell culture under sparging conditions. Biotechnology and bioengineering, Vol. 83, No. 1, July 5, 2003
Yallop C, Crowley J, Cote J, Hegmans-Brouwer K, Lagerwerf F, Gagne R, Martin JC, Oosterhuis N, Opstelten DJ, Bout A. Per.C6 cells for the manufacture of biopharmaceutical proteins. Modem Biophatmaceuticals - Design, Development and Optimization. Vol. 3, 2005
Yuk IHY, Olsen MM, Geyer S, Forestell SP. Perfusion Cultures of Human Tumor Cells: A Scalable Production Platform for Oncolytic Adenoviral Vectors. Biotechnol. Bioengin. 86: 637-641 (2004).

## Claims

1. A method for producing recombinant adenovirus serotype 26 (rAd26), the method comprising:
a) culturing PER.C6 cells in suspension with a perfusion system;
b) infecting said cells at a density of between 10x10⁶ viable cells/mL and 16x10⁶ viable cells/mL with rAd26;
c) further culturing the infected cells with a perfusion system to propagate said rAd26; and
d) harvesting said rAd26.

2. A method according to claim 1, wherein said cells in step b) are infected with rAd26 at a density of between about 10x10⁶ and 14x10⁶ viable cells/mL.

3. A method according to any one of the preceding claims, wherein said perfusion system in step c) is an alternating tangential flow (ATF) perfusion system.

4. A method according to any one of the preceding claims, further comprising:
e) purifying the rAd26, and optionally
f) preparing a pharmaceutical composition containing the purified rAd26.

5. A method according to any one of the preceding claims, wherein said recombinant adenovirus lacks at least a portion of the E1 region, and comprises heterologous nucleic acid.

6. A method according to any one of the preceding claims, wherein said perfusion system in step a) is an alternating tangential flow (ATF) perfusion system.

7. A method according to any one of the preceding claims, wherein step a) is performed in a first bioreactor, and steps b) and c) are performed in a second bioreactor.

8. A method according to any one of the preceding claims, wherein the physical particle to infectious particle (VP/IU) ratio of the produced rAd26 is less than 30:1, preferably less than 20:1.

9. Use of a bioreactor in a method according to any one of claims 1-8, wherein said bioreactor has a working volume of between 2L and 1000L, preferably between 50L and 500L, and comprises culture medium, PER.C6 cells, and at least 1x10¹² rAd26 virus particles (VP)/mL.

10. Use according to claim 9, wherein said bioreactor is connected to an ATF perfusion system.

11. Use according to claim 9 or 10, wherein the rAd26 virus particles have a VP/IU ratio of less than 30:1, preferably less than 20:1.

12. A method according to claim 1, wherein at least 1x10¹² rAd26 virus particles (VP)/mL are produced.

## Patentansprüche

1. Verfahren zur Herstellung eines rekombinanten Adenovirus Serotyp 26 (rAd26), wobei man bei dem Verfahren:
a) PER.C6-Zellen in Suspension mit einem Perfusionssystem kultiviert;
b) die Zellen bei einer Dichte zwischen 10x10⁶ lebensfähigen Zellen pro ml und 16x10⁶ lebensfähigen Zellen pro ml mit rAd26 infiziert;
c) die infizierten Zellen mit einem Perfusionssystem weiter kultiviert, um das rAd26 zu propagieren; und
d) das rAd26 erntet.

2. Verfahren nach Anspruch 1, wobei die Zellen in Schritt b) mit rAd26 bei einer Dichte zwischen etwa 10x10⁶ und 14x10⁶ lebensfähigen Zellen pro ml infiziert werden.

3. Verfahren nach einem der vorhergehenden Ansprüche, wobei es sich bei dem Perfusionssystem in Schritt c) um ein ATF(Alternating Tangential Flow)-Perfusionssystem handelt.

4. Verfahren nach einem der vorhergehenden Ansprüche, bei dem man ferner:
e) das rAd26 aufreinigt und gegebenenfalls
f) eine das aufgereinigte rAd26 enthaltende pharmazeutische Zusammensetzung zubereitet.

5. Verfahren nach einem der vorhergehenden Ansprüche, wobei dem rekombinanten Adenovirus wenigstens ein Teil der E1-Region fehlt und es heterologe Nukleinsäure umfasst.

6. Verfahren nach einem der vorhergehenden Ansprüche, wobei es sich bei dem Perfusionssystem in Schritt a) um ein ATF(Alternating Tangential Flow)-Perfusionssystem handelt.

7. Verfahren nach einem der vorhergehenden Ansprüche, wobei Schritt a) in einem ersten Bioreaktor und Schritt b) und c) in einem zweiten Bioreaktor durchgeführt werden.

8. Verfahren nach einem der vorhergehenden Ansprüche, wobei das Verhältnis von physikalischen zu infektiösen Partikeln (VP:IU-Verhältnis) des produzierten rAd26 bei weniger als 30:1, vorzugsweise weniger als 20:1 liegt.

9. Verwendung eines Bioreaktors bei einem Verfahren nach einem der Ansprüche 1 bis 8, wobei der Bioreaktor ein Arbeitsvolumen zwischen 2 1 und 1000 1, vorzugsweise zwischen 50 1 und 500 1, aufweist und Kulturmedium, PER.C6-Zellen, und wenigstens 1x10¹² rAd26-Viruspartikel (VP) pro ml umfasst.

10. Verwendung nach Anspruch 9, wobei der Bioreaktor mit einem ATF-Perfusionssystem verbunden ist.

11. Verwendung nach Anspruch 9 oder 10, wobei die rAd26-Viruspartikel ein VP/IU-Verhältnis von weniger als 30:1, vorzugsweise weniger als 20:1, aufweisen.

12. Verfahren nach Anspruch 1, wobei wenigstens 1x10¹² rAd26-Viruspartikel (VP) pro ml produziert werden.

## Revendications

1. Procédé de production d'un adénovirus de sérotype 26 recombinant (rAd26), le procédé comprenant les étapes consistant à :
a) cultiver des cellules PER.C6 en suspension avec un système de perfusion ;
b) infecter lesdites cellules, à une densité comprise entre 10 x 10⁶ cellules viables/mL et 16 x 10⁶ cellules viables/mL, avec un rAd26 ;
c) cultiver davantage les cellules infectées avec un système de perfusion afin de propager ledit rAd26 ; et
d) récolter ledit rAd26.

2. Procédé selon la revendication 1, dans laquelle lesdites cellules dans l'étape b) sont infectées avec un rAd26 à une densité comprise entre environ 10 x 10⁶ et 14 x 10⁶ cellules viables/mL.

3. Procédé selon l'une quelconque des revendications précédentes, dans laquelle ledit système de perfusion dans l'étape c) est un système de perfusion à flux tangentiel alternatif (FTA).

4. Procédé, selon l'une quelconque des revendications précédentes, comprenant en outre les étapes consistant à :
e) purifier le rAd26 et, en option,
f) préparer une composition pharmaceutique contenant le rAd26 purifié.

5. Procédé selon l'une quelconque des revendications précédentes, dans laquelle ledit adénovirus recombinant est dépourvu d'au moins une partie de la région E1 et comprend un acide nucléique hétérologue.

6. Procédé selon l'une quelconque des revendications précédentes, dans laquelle ledit système de perfusion dans l'étape a) est un système de perfusion à flux tangentiel alternatif (FTA).

7. Procédé selon l'une quelconque des revendications précédentes, dans laquelle l'étape a) est exécutée dans un premier bioréacteur et les étapes b) et c) sont exécutées dans un deuxième bioréacteur.

8. Procédé selon l'une quelconque des revendications précédentes, dans laquelle le rapport d'une particule physique à une particule infectieuse (VP/IU) du rAd26 produit est inférieur à 30:1, de préférence inférieur à 20:1.

9. Utilisation d'un bioréacteur dans un procédé selon l'une quelconque des revendications 1 à 8, dans laquelle ledit bioréacteur a un volume de travail compris entre 2L et 1 000L, de préférence entre 50L et 500L, et il comprend un milieu de culture, des cellules PER.C6 et au moins 1 x 10¹² particules de virus rAd26 (VP)/mL.

10. Utilisation selon la revendication 9, dans laquelle ledit bioréacteur est connecté à un système de perfusion à FTA.

11. Utilisation selon la revendication 9 ou la 10, dans laquelle les particules de virus rAd26 ont un rapport VP/IU inférieur à 30:1, de préférence inférieur à 20:1.

12. Procédé selon la revendication 1, dans laquelle au moins 1 x 10¹² particules de virus rAd26 (VP)/mL sont produites.
